# FASCICULE DE BREVET EUROPEEN

(11) **EP 1 682 056 B1**
(45) Date de publication et mention de la délivrance du brevet: **02.04.2008**
(21) Numéro de dépôt: 04805299.7
(22) Date de dépôt: 25.10.2004
(51) Int. Cl.: A61F 9/00

(54) **ENSEMBLE DE CONDITIONNEMENT ET DE DISTRIBUTION DE LIQUIDE A MEMBRANES MICROFILTRANTES**
ANORDNUNG ZUR VERPACKUNG UND ABGABE EINER FLÜSSIGKEIT MIT MEMBRAN-MIKROFILTERN
ASSEMBLY FOR PACKAGING AND DISPENSING A LIQUID, COMPRISING MEMBRANE MICROFILTERS

(30) Priorité: 10.11.2003 FR 0313200
(43) Date de publication de la demande: 26.07.2006
(73) Titulaire: Rexam Pharma, 76550 Offranville (FR)
(72) Inventeur: PETITDEMANGE, Jean, F-76730 AUPPERGARD (FR); GOSSEDGE, Graham, OXON Aberdeenshire OX11 OBS (GB)
(74) Mandataire: Domenego, Bertrand
(86) Numéro de dépôt international: PCT/FR2004/002740
(87) Numéro de publication internationale: WO 2005/046541

(56) Documents cités:
- EP-A- 0 401 022
- EP-A- 0 602 019
- WO-A-94/22412
- FR-A- 2 816 600
- US-A- 4 938 389
- US-A- 5 265 770

## Description

La présente invention concerne un ensemble de conditionnement et de distribution de liquide, du type comprenant :
- un récipient destiné à contenir le liquide, le récipient présentant un col s'étendant le long d'un axe longitudinal, et
- un dispositif de distribution de liquide, le dispositif de distribution étant disposé dans le col et comprenant :
   - un embout présentant un passage d'entrée d'air et de sortie de liquide qui comprend un tronçon aval par lequel il débouche à l'extérieur de l'ensemble, et
   - au moins une membrane microfiltrante liquophile et au moins une membrane microfiltrante liquophobe par l'intermédiaire desquelles le passage communique avec l'intérieur du récipient.

L'invention s'applique en particulier, mais pas exclusivement, à la distribution de collyres.

La stérilité de tels liquides doit être garantie.

Pendant longtemps, cette stérilité a été assurée en ajoutant des conservateurs au collyre. Ainsi, les agents contaminants provenant de l'extérieur, tels que des bactéries, pouvaient pénétrer dans le récipient contenant le collyre, sans pour autant en affecter la stérilité.

Toutefois, il s'est avéré que de nombreuses personnes présentent des allergies à ces conservateurs.

Pour pallier cet inconvénient, GB-2 021 429 et, par exemple, EP-401 022 ont proposé des ensembles du type précité. Dans de tels ensembles, les membranes liquophile et liquophobe sont disposées l'une à côté de l'autre, dans un plan transversal à l'axe longitudinal du col du récipient. Lors de la distribution du liquide, celui-ci traverse longitudinalement la membrane liquophile. Cette membrane retient les agents contaminants qui auraient pu pénétrer à l'intérieur du récipient. Le collyre distribué est donc stérile.

L'air aspiré à l'intérieur du récipient suite à la distribution du liquide est quant à lui filtré par la membrane liquophobe pour empêcher l'entrée d'agents contaminants dans le récipient.

De tels ensembles permettent d'éviter l'emploi de conservateurs.

EP-401 022 enseigne également que, pour éviter la formation d'un jet de liquide, et ainsi garantir l'obtention d'une distribution goutte à goutte, la surface active de la membrane liquophile, c'est-à-dire la surface traversée par le liquide, doit être faible. Cependant, l'emploi d'une membrane liquophile de faible surface active nécessite d'appliquer des efforts importants sur le récipient afin de distribuer le liquide. Cette solution n'est donc pas satisfaisante pour garantir l'obtention d'une distribution goutte à goutte.

Par ailleurs, l'utilisation de membranes liquophiles de surfaces plus importantes conduirait, avec la configuration du document EP-401 022, à des dispositifs de distribution très larges et à des ensembles de conditionnement et de distribution très encombrants.

Un but de l'invention est de résoudre ce problème en fournissant un ensemble du type précité qui permette de limiter la formation d'un jet de liquide, sans qu'il soit nécessaire d'exercer une pression très importante pour distribuer le liquide et tout en conservant un encombrement réduit.

A cet effet, l'invention a pour objet un ensemble du type précité, caractérisé en ce que la membrane liquophile s'étend au moins le long de l'axe longitudinal du col du flacon.

Selon des modes particuliers de réalisation, l'ensemble peut comprendre l'un ou plusieurs des caractéristiques suivantes prise(s) isolément ou selon toutes les combinaisons techniquement possibles :
- la membrane liquophile appartient sensiblement à une surface géométrique de révolution autour de l'axe longitudinal,
- la membrane liquophobe appartient à une paroi de forme adaptée pour que du liquide présent sur la membrane liquophobe en soit évacué sous l'effet de la gravité,
- la membrane liquophile est portée par une paroi de l'embout qui délimite le passage d'entrée d'air et de sortie de liquide, et la membrane liquophobe est portée par un insert logé dans le passage d'entrée d'air et de sortie de liquide,
- la membrane liquophobe est disposée à l'extrémité supérieure de l'insert,
- l'insert comprend une paroi latérale inclinée vers le bas et vers la membrane liquophile pour renvoyer, sous l'effet de la gravité, du liquide présent dans le passage au travers de la membrane liquophile vers l'intérieur du récipient,
- la membrane liquophile a une surface active supérieure à 50 mm²,
- la membrane liquophile a une surface active supérieure à 100 mm²,
- la membrane liquophile et la membrane liquophobe comprennent des pores de dimensions inférieures à 0,2 µm,
- un liquide dépourvu de conservateur est contenu dans le récipient,
- le passage d'entrée d'air et de sortie de liquide débouche à l'extérieur de l'ensemble par un tronçon unique.

L'invention sera mieux comprise à la lecture de la description qui va suivre, donnée uniquement à titre d'exemple, et faite en se référant aux dessins annexés, sur lesquels :
- la figure 1 est une vue schématique, agrandie et en coupe longitudinale d'un ensemble de conditionnement et de distribution de liquide selon l'invention,
- la figure 2 est une vue schématique en perspective du dispositif de distribution de l'ensemble de la figure 1,
- la figure 3 est une coupe schématique brisée à plans séquents de l'embout du dispositif de distribution de la figure 2, la trace des deux plans sécants étant matérialisée par la ligne brisée III-III sur la figure 2,
- la figure 4 est une vue schématique en perspective de l'insert du dispositif de distribution de la figure 2,
- la figure 5 est une vue schématique en coupe longitudinale de l'insert de la figure 4, et
- la figure 6 est une vue schématique en coupe longitudinale de l'insert dans un plan VI-VI orthogonal au plan de la figure 5.

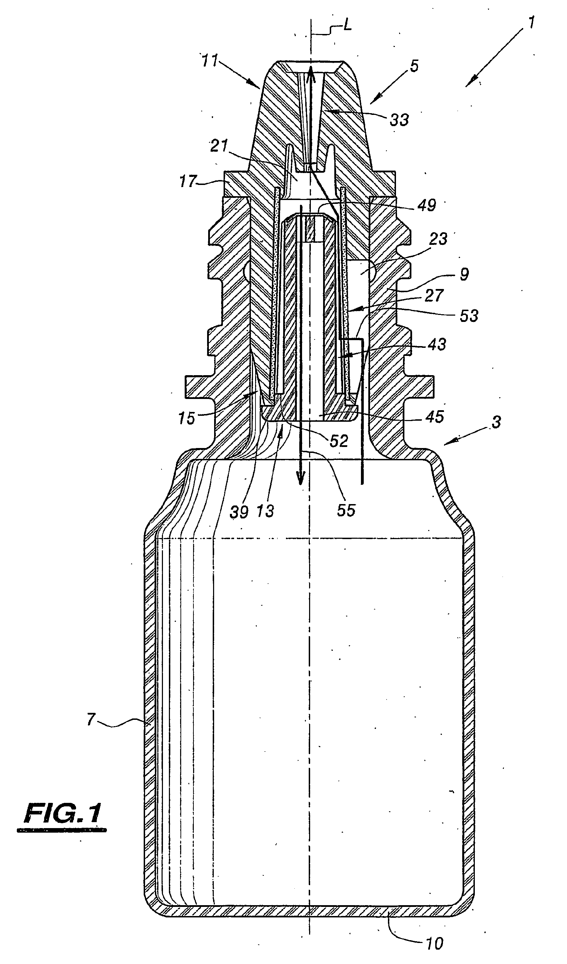

La figure 1 illustre schématiquement un ensemble 1 de conditionnement et de distribution d'un collyre sous forme d'une solution aqueuse.

Cet ensemble 1 comprend un récipient 3 et un dispositif 5 de distribution du collyre.

Le récipient 3 est dans l'exemple représenté un flacon en matière plastique. Ce flacon 3 contient le collyre qui n'a pas été représenté sur les figures.

Le récipient 3 comprend un corps 7 prolongé par un col supérieur 9 qui s'étend le long d'un axe longitudinal L vertical.

De manière classique, les parois du corps 7 sont déformables élastiquement sous l'action d'un effort transversal pour provoquer une diminution du volume interne du corps 7 et ainsi permettre l'expulsion du collyre au travers du col 9. Le récipient 3 peut reposer sur une surface d'appui par l'intermédiaire du fond 10 du corps 7.

De manière classique également, le col 9 est muni de reliefs extérieurs tels que des filets, permettant la liaison à un bouchon de fermeture non-représenté.

Ce bouchon, qui sera enlevé avant utilisation de l'ensemble 1 et replacé sur le col 9 après utilisation, peut par exemple comprendre des moyens d'inviolabilité permettant de s'assurer, lors de la première utilisation, que l'ensemble 1 n'a pas été utilisé auparavant.

Le dispositif de distribution 5 comprend un embout 11 enfiché dans le col 9 et un insert 13 lui-même disposé à l'intérieur de l'embout 11.

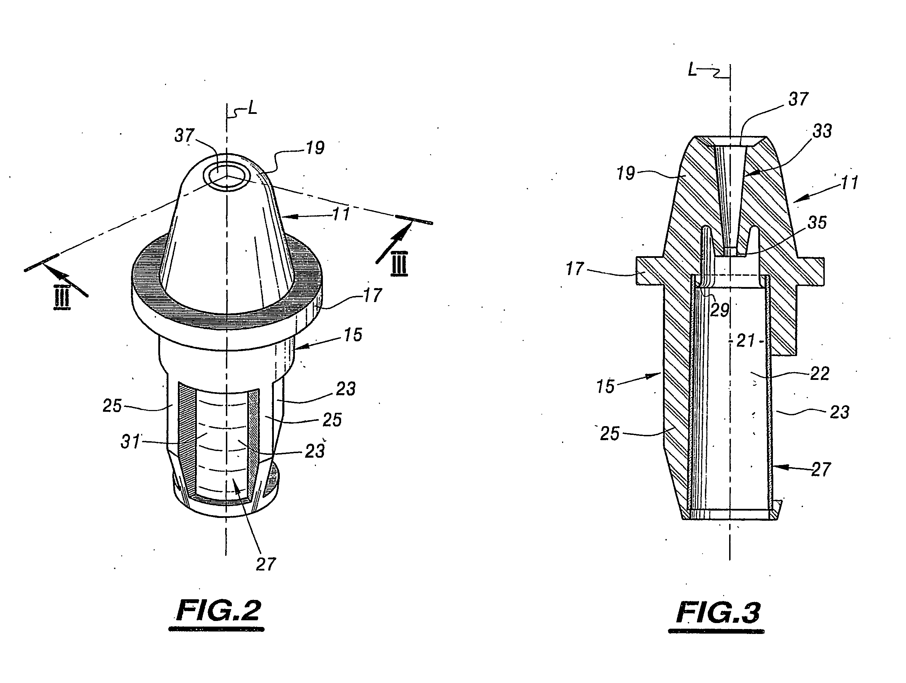

Comme illustré par les figures 2 et 3, l'embout 11 comprend une extrémité inférieure 15, une collerette intermédiaire 17 et une pointe compte-gouttes supérieure 19. Un passage longitudinal 21 traverse l'embout 11 de part en part.

Le tronçon inférieur 22 du passage 21 a sensiblement une forme cylindrique centrée sur l'axe longitudinal L. Quatre fenêtres 23 sont ménagées dans la paroi latérale de l'extrémité inférieure 15 pour faire communiquer le tronçon inférieur 22 du passage 21 transversalement avec l'extérieur de l'embout 11.

Ces fenêtres 23 sont séparées latéralement les unes des autres par des bandes longitudinales 25 de la paroi de l'extrémité 15. Les fenêtres 23 sont réparties régulièrement angulairement autour de l'axe L, l'embout 11 ayant une forme générale de révolution autour de l'axe L.

Une membrane hydrophile 27 est disposée dans le tronçon inférieur 22 du passage 21, c'est-à-dire dans l'extrémité inférieure 15 de l'embout 11. II s'agit d'une membrane microfiltrante présentant des pores de dimensions de préférence inférieures à 0,2µm.

Cette membrane hydrophile 27 a une forme sensiblement cylindrique centrée sur l'axe longitudinal L. En variante, elle peut avoir par exemple une forme convergeant légèrement vers le haut. La membrane 27 prend appui sur la paroi intérieure délimitant le tronçon inférieur 22 du passage 21, et notamment sur les bandes 25.

L'extrémité supérieure de la membrane 27 est engagée sous un rebord circulaire 29 de l'embout 11.

L'embout 11 a par exemple été réalisé par surmoulage sur la membrane 27, assurant ainsi la solidarisation de la membrane 27 et de l'embout 11.

Comme on peut le voir sur l'exemple des figures 2 et 3, quatre régions 31 de la membrane 27 sont exposées au travers des fenêtres 23. Ces régions 31 ont chacune une forme développée rectangulaire. En variante, le nombre de régions 31 peut être différent de quatre.

Ces régions 31, par l'intermédiaire desquelles le tronçon inférieur 22 du passage 21 communique avec l'extérieur de l'embout 11, forment des zones microfiltrantes. La somme totale des surfaces des régions 31 est par exemple supérieure à 50 mm², et de préférence supérieure à 100 mm². Cette somme est dénommée surface active de la membrane 27 puisque c'est celle qui sera traversée par le collyre lors de la distribution, comme cela sera exposé par la suite.

Le tronçon supérieur ou aval 33 du passage 21, qui est ménagé dans la pointe 19, communique par un orifice inférieur ou amont 35 avec le reste du passage 21, et débouche à l'extérieur de la pointe 19 par un orifice supérieur ou aval 37.

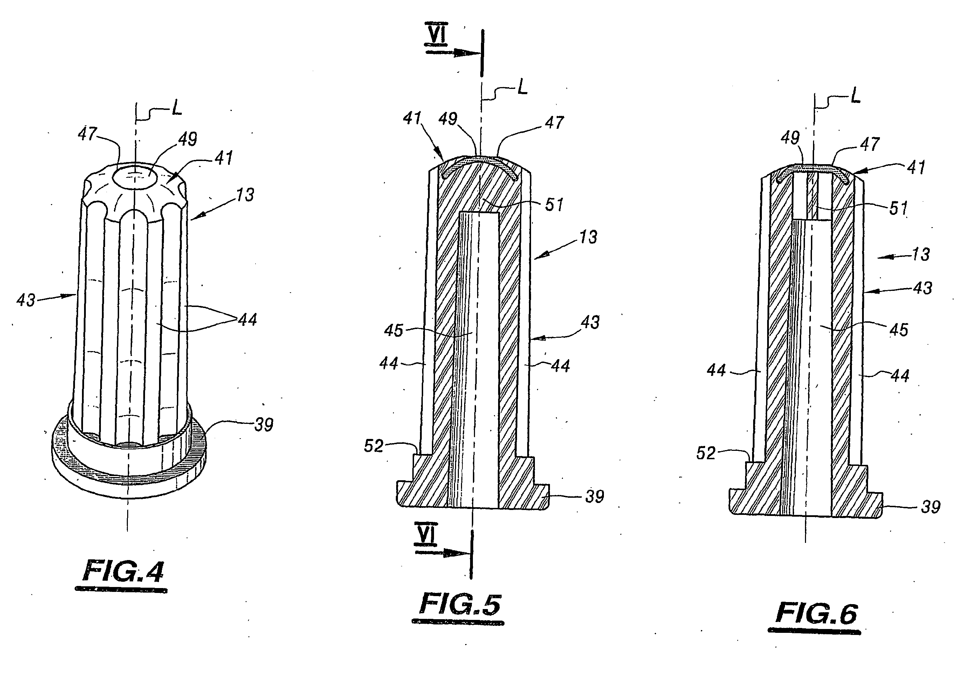

L'insert 13 a, comme illustré par les figures 4 à 6, une forme de manchon creux s'étendant le long de l'axe longitudinal L, prolongé à son extrémité inférieure par une collerette 39 et fermé à son extrémité supérieure par une paroi transversale 41 bombée et convexe.

La paroi latérale 43 de l'insert 13 a une forme convergeant légèrement vers le haut le long de l'axe L.

Cette paroi latérale 43 présente des nervures longitudinales 44 espacées angulairement les unes des autres autour de l'axe longitudinal L. Un canal 45 traverse longitudinalement l'insert 13 et débouche dans la paroi supérieure 41 par une ouverture 47.

Les dimensions du canal 45 sont de préférence faibles pour que peu de collyre puisse y pénétrer lorsque l'ensemble 1 est retourné.

Dans une variante non-représentée, l'extrémité inférieure du canal 45 peut représenter des chicanes en vue de limiter l'entrée de collyre dans le canal 45.

Une membrane hydrophobe 49 est disposée dans la paroi 41 et s'étend notamment au travers de l'ouverture supérieure 47. La membrane 49 est une membrane microfiltrante dont les pores ont de préférence des dimensions inférieures à 0,2 µm.

Une nervure transversale 51 est prévue dans l'extrémité supérieure du canal 45 sous la membrane 49.

Cette nervure 51 supporte la membrane 49, comme on le voit sur la figure 5. Comme on le voit sur la figure 6, cette nervure 51 n'obture pas complètement le canal 45. L'insert 13 a par exemple été surmoulé sur la membrane 49 en assurant ainsi leur solidarisation.

Comme on le voit sur les figures 1 et 2, l'insert 13 est emmanché dans le tronçon inférieur 22 du passage 21 de l'embout 11. La collerette inférieure 39 de l'insert 13 est appuyée contre l'extrémité inférieure 15 de l'embout 11 en assurant une étanchéité à l'air.

A titre d'exemple, cette étanchéité peut être assurée par simple interférence ou par soudage de la collerette 39 sur l'extrémité 15 par exemple par ultrasons ou par friction.

On notera qu'un épaulement 52 de la paroi latérale 43 de l'insert 13 vient au contact de l'extrémité inférieure des régions 31 de la membrane hydrophile 27.

Dans le dispositif de distribution 5, l'embout 11 et l'insert 13 sont donc assemblés l'un à l'autre.

Le dispositif 5 a été en outre assemblé au flacon 3 par emmanchement de la partie inférieure 15 de l'embout 11 dans le col 9, la collerette 17 s'appuyant sur la tranche du col 9. La liaison entre l'embout 11 et le flacon 3 est également étanche à l'air. Cette étanchéité peut être assurée par simple interférence ou par soudage de la collerette 17 sur le col 9 par exemple par ultrasons ou par friction.

En outre, un agent bactériostatique est éventuellement présent sur les surfaces extérieures de l'ensemble 1, par exemple par l'intermédiaire d'éléments rapportés sur le flacon 3.

De manière classique, un tel agent peut contenir un composé à base d'argent, pour détruire les microorganismes.

Pour utiliser l'ensemble 1, on procède comme suit.

Après avoir préalablement ôté le bouchon de fermeture non-représenté, on retourne l'ensemble 1 et on presse les parois latérales du corps 7 du flacon 3 de manière à accroître la pression à l'intérieur de ce dernier, ce qui tend à expulser le collyre hors du corps 7 au travers du col 9.

Comme matérialisé par la flèche 53 sur la figure 1, le collyre entre alors au contact de la membrane hydrophile 27 au travers des fenêtres 23 de l'embout 11. Le collyre traverse alors transversalement la membrane 27 en étant filtré puis est renvoyé longitudinalement le long de la paroi latérale 43 de l'insert 13 vers le haut. Le collyre pénètre ensuite dans le tronçon aval 33 du passage 21 avant d'être distribué longitudinalement par la pointe compte-gouttes 19.

On notera que si du collyre a pénétré dans le canal 45 de l'insert 13, il est bloqué par la membrane 49 qui est hydrophobe et empêche donc son passage.

La membrane 27 assure ainsi la distribution d'un collyre stérile en retenant dans le récipient 3 les bactéries et autres agents contaminants qu'il pourrait contenir.

En outre, le collyre s'écoulant transversalement au travers de la membrane 27 avant d'être réorienté pour s'écouler longitudinalement dans le tronçon aval 33, le risque qu'un jet longitudinal de collyre se produise à la sortie de la pointe compte-gouttes 19 est réduit.

Lorsque la pression exercée sur les parois latérales du corps 7 du flacon 3 est relâchée, le corps 7 reprend sa forme initiale en créant une dépression au travers des membranes 27 et 49.

La membrane 27 étant hydrophile et étant mouillée par du collyre, elle empêche le passage d'air.

En revanche, l'entrée d'air dans le flacon 3 peut s'effectuer, au travers de la membrane hydrophobe 49 puis via le canal 45. Cela est matérialisé par la flèche 55 sur la figure 1.

La membrane hydrophobe 49 empêche ainsi l'entrée d'agents contaminants à l'intérieur du flacon 3.

Lorsque l'on retourne l'ensemble pour qu'il reprenne sa position représentée sur la figure 1, avec le dispositif de distribution 5 orienté vers le haut, le collyre qui pourrait se trouver sur la paroi 41 est, du fait de la forme convexe de cette paroi, renvoyé vers la paroi latérale 43 de l'insert 13.

Cette paroi 43 divergeant vers le bas et vers la membrane hydrophile 27, le collyre provenant de la paroi 41 ainsi que celui qui aurait pu franchir la membrane 27 et se trouverait à l'intérieur du passage 21 est alors renvoyé par gravité vers la membrane 27 et revient à l'intérieur du flacon 3.

La membrane 27 empêche que ce retour du collyre soit accompagné de l'entrée d'agents contaminants.

La paroi latérale 43 rejoignant via l'épaulement 52 l'extrémité inférieure des régions 31 de la membrane 27, il n'y a pas de risque d'accumulation de collyre dans une zone située sous ces régions 31, d'où il ne pourrait être renvoyé vers l'intérieur du flacon 3.

L'ensemble 1 permet donc de conditionner et de distribuer un collyre stérile sans que l'utilisation de conservateur dans le collyre ne soit nécessaire.

En outre, la membrane hydrophile 27 s'étendant avec au moins une composante longitudinale, sa surface active peut être importante tout en conservant un encombrement réduit pour l'ensemble 1.

Cela est d'autant plus vrai que d'une part, la membrane 27 a sensiblement une forme de révolution autour de l'axe L et d'autre part que l'intérieur du flacon 3 communique avec l'extérieur par l'intermédiaire d'un unique tronçon aval 33 et d'un unique orifice aval 37 d'entrée d'air et de sortie de collyre.

De manière générale, la surface active de la membrane 27 peut être différente de celles citées précédemment et être inférieure à 50mm².

Comme indiqué précédemment, la membrane 27 s'étendant longitudinalement, l'écoulement du collyre a au moins une composante transversale à la traversée de la membrane 27, limitant ainsi les risques de formation d'un jet longitudinal en aval. Dans une variante le tronçon 33 peut avoir en outre une forme adaptée pour empêcher la formation d'un jet de liquide de sorte que la distribution du collyre par la pointe 19 s'effectue nécessairement goutte à goutte. De telles formes sont connues et ne seront pas décrites plus en détail.

De manière plus générale, la membrane 27 peut avoir une forme autre que cylindrique et peut par exemple comprendre plusieurs tronçons qui appartiennent à une même surface de révolution. En particulier, la membrane 27 peut avoir des tronçons inclinés par rapport à l'axe L.

Même si l'invention est particulièrement destinée à la distribution de produits pharmaceutiques, elle peut être utilisée de manière générale dans tous les domaines requérant la distribution de liquides stériles.

Ainsi, les membranes 27 et 49 peuvent être plus généralement des membranes respectivement liquophile et liquophobe.

## Revendications

1. Ensemble (1) de conditionnement et de distribution de liquide, du type comprenant :
- un récipient (3) destiné à contenir le liquide, le récipient (3) présentant un col (9) s'étendant le long d'un axe longitudinal (L), et
- un dispositif (5) de distribution de liquide, le dispositif de distribution (5) étant disposé dans le col (9) et comprenant:
. un embout (11) présentant un passage (21) d'entrée d'air et de sortie de liquide qui comprend un tronçon aval (33) par lequel il débouche à l'extérieur de l'ensemble (1), et
. au moins une membrane microfiltrante liquophile (27) et au moins une membrane microfiltrante liquophobe (49) par l'intermédiaire desquelles le passage (21) communique avec l'intérieur du récipient (3),
**caractérisé en ce que** la membrane liquophile (27) s'étend au moins le long de l'axe longitudinal (L) du col (9) du flacon.

2. Ensemble selon la revendication 1, **caractérisé en ce que** la membrane liquophile (27) appartient sensiblement à une surface géométrique de révolution autour de l'axe longitudinal (L).

3. Ensemble selon l'une des revendications précédentes, **caractérisé en ce que** la membrane liquophobe (49) appartient à une paroi (41) de forme adaptée pour que du liquide présent sur la membrane liquophobe (49) en soit évacué sous l'effet de la gravité.

4. Ensemble selon l'une des revendications précédentes, **caractérisé en ce que** la membrane liquophile (27) est portée par une paroi de l'embout (11) qui délimite le passage (21) d'entrée d'air et de sortie de liquide, et **en ce que** la membrane liquophobe (49) est portée par un insert (13) logé dans le passage (21) d'entrée d'air et de sortie de liquide.

5. Ensemble selon les revendications 3 et 4 prises ensemble, **caractérisé en ce que** la membrane liquophobe (49) est disposée à l'extrémité supérieure de l'insert (13).

6. Ensemble selon la revendication 4 ou 5, **caractérisé en ce que** l'insert (13) comprend une paroi latérale (43) inclinée vers le bas et vers la membrane liquophile (27) pour renvoyer, sous l'effet de la gravité, du liquide présent dans le passage (21) au travers de la membrane liquophile (27) vers l'intérieur du récipient (3).

7. Ensemble selon l'une des revendications précédentes, **caractérisé en ce que** la membrane liquophile (27) a une surface active supérieure à 50 mm².

8. Ensemble selon la revendication 7, **caractérisé en ce que** la membrane liquophile (27) a une surface active supérieure à 100 mm².

9. Ensemble selon l'une des revendications précédentes, **caractérisé en ce que** la membrane liquophile (27) et la membrane liquophobe (49) comprennent des pores de dimensions inférieures à 0,2 µm.

10. Ensemble selon l'une des revendications précédentes, **caractérisé en ce qu'**un liquide dépourvu de conservateur est contenu dans le récipient (3).

11. Ensemble selon l'une des revendications précédentes, **caractérisé en ce que** le passage (21) d'entrée d'air et de sortie de liquide débouche à l'extérieur de l'ensemble (1) par un tronçon unique (33).

## Claims

1. An assembly (1) for packaging and distributing liquid of the type comprising:
- a receptacle (3) intended to contain the liquid, the receptacle (3) having a neck (9) extending along a longitudinal axis (L), and
- a liquid distribution device (5), the distribution device (5) being arranged in the neck (9) and comprising:
. a nozzle (11) having an air inlet and liquid outlet passage (21) which comprises a downstream section (33) by means of which it opens to the exterior of the assembly (1), and
. at least one liquophilic microfiltration membrane (27) and at least one liquophobic microfiltration membrane (49) by means of which the passage (21) communicates with the interior of the receptacle (3),
**characterised in that** the liquophilic membrane (27) extends at least along the longitudinal axis (L) of the neck (9) of the bottle.

2. An assembly according to claim 1, **characterised in that** the liquophilic membrane (27) substantially belongs to a geometric surface of revolution about the longitudinal axis (L).

3. An assembly according to any one of the preceding claims, **characterised in that** the liquophobic membrane (49) belongs to a wall (41) of a shape which is such that any liquid present on the liquophobic membrane (49) is removed therefrom under the effect of gravity.

4. An assembly according to any one of the preceding claims, **characterised in that** the liquophilic membrane (27) is borne by a wall of the nozzle (11) which defines the air inlet and liquid outlet passage (21), and **in that** the liquophobic membrane (49) is borne by an insert (13) accommodated in the air inlet and liquid outlet passage (21).

5. An assembly according to claims 3 and 4 taken together, **characterised in that** the liquophobic membrane (49) is arranged at the upper end of the insert (13).

6. An assembly according to claim 4 or claim 5,
**characterised in that** the insert (13) comprises a lateral wall (43) inclined downwards and towards the liquophilic membrane (27) in order to return, under the action of gravity, any liquid present in the passage (21) through the liquophilic membrane (27) towards the interior of the receptacle (3).

7. An assembly according to any one of the preceding claims, **characterised in that** the liquophilic membrane (27) has an active surface area of greater than 50 mm².

8. An assembly according to claim 7, **characterised in that** the liquophilic membrane (27) has an active surface area of greater than 100 mm².

9. An assembly according to any one of the preceding claims, **characterised in that** the liquophilic membrane (27) and the liquophobic membrane (49) comprise pores of dimensions of less than 0.2 µm.

10. An assembly according to any one of the preceding claims, **characterised in that** a liquid containing no preservative is contained in the receptacle (3).

11. An assembly according to any one of the preceding claims, **characterised in that** the air inlet and liquid outlet passage (21) opens to the exterior of the assembly (1) via a single section (33).

## Patentansprüche

1. Aufbau (1) zur Verpackung und Abgabe von Flüssigkeit, wobei dieser aufweist:
einen Behälter (3) zur Aufnahme von Flüssigkeit, wobei der Behälter (3) einen Hals (9) aufweist, der sich längs einer Längsachse (L) erstreckt, und
eine Vorrichtung (5) zur Abgabe von Flüssigkeit, wobei die Abgabevorrichtung (5) im Hals (9) angeordnet ist und aufweist:
ein Endstück (11), welches einen Lufteintritts- und Flüssigkeitsaustrittsdurchgang (21) aufweist, welcher einen stromabwärts liegenden Abschnitt (33) aufweist, mit welchem er außerhalb des Aufbaus (1) mündet, und
wenigstens eine liquophile Mikrofilter-Membran (27) und wenigstens eine liquophobe Mikrofilter-Membran (49), über welche der Durchgang (21) mit dem Inneren des Behälters (3) in Verbindung steht,
**dadurch gekennzeichnet, dass** die liquophile Membran (27) sich wenigstens längs der Längsachse (L) des Halses (9) der Flasche erstreckt.

2. Aufbau nach Anspruch 1, **dadurch gekennzeichnet, dass** die liquophile Membran (27) im Wesentlichen einer geometrischen Rotationsfläche um die Längsachse (L) zugehörig ist.

3. Aufbau nach einem der vorstehenden Ansprüche, **dadurch gekennzeichnet, dass** die liquophobe Membran (49) im Wesentlichen einer Wand (41) mit einer Form zugehörig ist, die so eingerichtet ist, dass auf der liquophoben Membran (49) vorhandene Flüssigkeit von dieser unter der Wirkung der Schwerkraft abgeführt wird.

4. Anordnung nach einem der vorstehenden Ansprüche, **dadurch gekennzeichnet, dass** die liquophile Membran (27) von einer Wand des Endstücks (11) getragen wird, welche den Lufteintritts- und Flüssigkeitsaustrittsdurchgang (21) begrenzt, und dass die liquophobe Membran (49) von einem Einsatz (13) getragen wird, der in den Lufteintritts- und Flüssigkeitsaustrittsdurchgang (21) eingesetzt ist.

5. Aufbau nach den Ansprüchen 3 und 4 zusammengenommen, **dadurch gekennzeichnet, dass** die liquophobe Membran (49) am oberen Ende des Einsatzes (13) angeordnet ist.

6. Aufbau nach Anspruch 4 oder 5, **dadurch gekennzeichnet, dass** der Einsatz (13) eine nach unten und zur liquophoben Membran (27) geneigte seitliche Wand (43) aufweist, um unter der Wirkung der Schwerkraft Flüssigkeit, die in dem Durchgang (21) vorhanden ist, durch die liquophile Membran (27) hindurch in das Innere des Behälters (3) zurückzuführen.

7. Aufbau nach einem der vorstehenden Ansprüche, **dadurch gekennzeichnet, dass** die liquophile Membran eine aktive Oberfläche von mehr als 50 mm² hat.

8. Aufbau nach Anspruch 7, **dadurch gekennzeichnet, dass** die liquophile Membran eine aktive Oberfläche von mehr als 100 mm² hat.

9. Aufbau nach einem der vorstehenden Ansprüche, **dadurch gekennzeichnet, dass** die liquophile Membran (27) und die liquophobe Membran (49) Poren mit Abmessungen kleiner als 0,2 µm aufweisen.

10. Aufbau nach einem der vorstehenden Ansprüche, **dadurch gekennzeichnet, dass** eine konservierungsstofffreie Flüssigkeit in dem Behälter (3) enthalten ist.

11. Aufbau nach einem der vorstehenden Ansprüche, **dadurch gekennzeichnet, dass** der Lufteintritts- und Flüssigkeitsaustrittsdurchgang (21) über einen einzelnen Abschnitt (33) außerhalb des Aufbaus (1) mündet.
